(19) Europäisches Patentamt
European Patent Office
Office européen des brevets

(11) Publication number: 0 093 522 B1

(12) EUROPEAN PATENT SPECIFICATION

(45) Date of publication of patent specification: 24.07.85

(51) Int. Cl.$^4$: C 07 C 76/00, C 07 C 79/04

(21) Application number: 83302092.8

(22) Date of filing: 14.04.83

# (54) Nitration of organic compounds and organic nitrogen compounds produced.

(30) Priority: 01.05.82 GB 8212952
21.09.82 GB 8226938

(43) Date of publication of application:
09.11.83 Bulletin 83/45

(45) Publication of the grant of the patent:
24.07.85 Bulletin 85/30

(84) Designated Contracting States:
BE DE FR GB IT NL SE

(56) References cited:
DE-A- 902 374
GB-A-2 000 141
US-A-2 609 401

INDUSTRIAL AND ENGINEERING CHEMISTRY, vol. 41, no. 10, October 1949, pages 2266-2270 H.B. HASS et al.: "Oxygen-induced vapor-phase nitration of paraffins"

(73) Proprietor: Interox Chemicals Limited
Hanover House 14 Hanover Square
London W1R 0BE (GB)

(72) Inventor: Campbell, Ian McIntyre
18, St. Chad's View
Leeds (GB)
Inventor: Baulch, Donald Leslie
52, Becketts Park Drive
Leeds (GB)
Inventor: Audley, Gary James
37, Hamble Walk
Goldsworth Park Woking Surrey (GB)
Inventor: Chappel, Jonathan Mark
78, Chestnut Avenue
Holbeach Spalding Lincolnshire (GB)

(74) Representative: ffrench-Lynch, Cecil et al
Laporte Industries Ltd., Group Patent
Department, P.O. Box 2, Moorfield Road
Widnes, Cheshire WA8 OJU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 093 522 B1

**Description**

This invention relates to the nitration of organic compounds and nitrogen compounds so produced. The term "nitration" is used to mean, in general sense, the inclusion of nitrogen containing groups in organic compounds.

The high temperature non-photolytic vapour phase nitration of hydrocarbons by means of nitric acid or nitrogen dioxide is referred to in the Encyclopaedia of Chemical Technology (Kirk-Othmer) Ed. 2 Vol. 13 page 790. That reference discloses that, when nitrogen dioxide is used as the nitrating agent, nitration occurs at temperatures of from about 200°C to 450°C. Pressures of 1 to 12 atmospheres (1 to 12×101325 $Nm^{-2}$) may be used although pressures above atmospheric are used commercially. At lower temperatures, within the disclosed range, a residence time of up to several minutes is required to obtain a significant degree of reaction and, even then, the conversion of nitrogen dioxide to the desired organic nitrogen compounds is significantly less than that obtained using nitric acid. An alternative process for the vapour phase nitration of hydrocarbons using nitrogen dioxide could therefore be of considerable commercial benefit in view of the above stated disadvantages.

A theoretical study of the reactivity of hydroxyl radicals towards a reactive substrate (butane) to produce a radical of the substrate (butyl) using the reaction between hydrogen peroxide and nitrogen dioxide as a source of hydroxyl radicals has been published in the Journal of the Chemical Society (Faraday Transactions I) 1975 Vol. 71 pages 867 to 874. In that study the reaction between the hydroxyl radical and the substrate was essentially conducted in the presence of an excess of carbon monoxide, within the range

$$\frac{[\text{butane}]}{[\text{CO}]}=0.005 \text{ to } 0.08$$

which carbon monoxide competed with the substrate for the hydroxyl radicals by virtue of the reaction

$$CO+OH \rightarrow CO_2+H.$$

The decrease in the yield of carbon dioxide in the presence of the substrate gives a measure of the reactivity of the substrate with hydroxyl radicals in comparison with that of carbon monoxide. Since the reactivity of carbon monoxide is known an estimate of the reactivity of the substrate with hydroxyl radicals may be derived. The theoretical possibility that some of the radicals of substrate could combine with nitrogen dioxide to give a low concentration of the corresponding nitrite is envisaged although no such product was actually confirmed to be present.

It has been found, according to the present invention, that the reaction between hydrogen peroxide and nitrogen dioxide may be used, in the absence of CO, as a source of hydroxyl radicals for organic synthesis. It has further been found, according to the present invention, that such a reaction may be used in a practical vapour phase process for the nitration of organic compounds. The process may be operated non-photolytically at or near ambient temperature and at ambient or reduced pressure using relatively short residence times. By 'non-photolytic' is meant a reaction in which no photon energy is used to break a bond. Only thermal reactions are involved herein which can achieve a higher energy efficiency overall than a photolytic process.

According to a particular aspect thereof the present invention provides a process for the nitration of one or more organic compounds comprising reacting in the vapour phase, the one or more organic compounds with a nitrating agent, the process being characterised in that radicals of the one or more organic compounds are produced for nitration by reacting hydrogen peroxide vapour and nitrogen dioxide in the presence of a solid surface to produce hydroxyl radicals and reacting the hydroxyl radicals with a vapour of a reactive substrate mainly comprising the one or more organic compounds by forming a mixture containing the hydroxyl radicals and the vapour of the reactive substrate or containing hydrogen peroxide vapour and nitrogen dioxide, as hydroxyl radical precursors, in the presence of the solid surface, and the vapour of the reactive substrate and in that the radicals so produced are contacted with the nitrating agent. The terminology "mainly comprising the one or more organic compounds" is used to limit to a minor proportion, that is to less than 50% on a molar basis, the presence of any other material and in particular CO, which is capable of reacting with the hydroxyl radicals in competition with the organic compound, thereby reducing the overall yield based on the quantity of hydrogen peroxide used. Preferably the minor proportion is less than 20%, particularly, preferably not more than 10%, on a molar basis.

The nitration of the organic radicals may be conducted using any suitable nitrating agent. However, it is found particularly advantageous for the nitration to be conducted "in situ" in a single process stage in the radical production reaction medium and in this case it is preferred that the nitrating agent is nitrogen dioxide, suitably included as an excess over that required to produce the hydroxyl radicals.

Subject to the consideration that the organic compound, to be suitable for use as a substrate in the present invention, must be vaporisable at the pressure and temperature employed and reactive with hydroxyl radicals, a wide variety of organic compounds may be employed as the substrate. Examples of aliphatic compounds from which suitable substrates may be selected are alcohols, ketones, aldehydes or

ethers, or preferably, alkanes. Aromatic compounds may be found to be suitable as may aromatic derivatives of aliphatic compounds. The presence on the organic compound of inorganic substituents which do not interfere with the process nor the suitability of the organic compound is not excluded.

The alkanes which may be straight or branched chain and, preferably contain from 1 to 20 carbon atoms and particularly preferably from 1 to 10 carbon atoms. Examples of particularly suitable alkanes are methane, ethane, n-butane and n-pentane.

It is found that, to increase the yield of hydroxyl radicals, the reaction between the hydrogen peroxide and the nitrogen dioxide to form the hydroxyl radicals is advantageously conducted in the presence of a solid surface suitable to encourage the adsorption of the hydrogen peroxide vapour thereon.

Preferably, such a surface is an acidic solid surface is used, such surface being found to have a catalytic effect on the reaction. Suitable acidic solid surfaces may be composed of an acid which may be a solid acid or a liquid or gaseous acid adsorbed on an inert surface. Preferably the acidic solid surface so provided by a solid inorganic acid such as, for example, boric acid or fused phosphoric acid. Alternatively, the acidic solid surface may be composed of or comprise one or more acidic oxides or mixed oxides, such as alumina, or a silica-alumina compound such as a zeolite or other silica containing acidic mixed oxide. In a paper in Industrial an Engineering Chemistry Nov. 1974 pages 2564 to 2573 there is postulated a test whereby acidity in oxides or mixed oxides may be detected. The test involves the measurement of the quantity of KOH which will react with a given weight of the oxide as indicated by titration with 0.1N HCl using phenolphthalein as indicator. The said test may be as a criterion for the selection of acidic oxides or mixed oxides for use according to this invention in which event, preferably, the acidity of the acidic solid surface material is at least 1.0 particularly preferably at least 2 meKOH/g. Suitably, the material providing the acidic surface is coated onto a suitable support such as a refractory reactor packing material to enhance the surface area available to the hydrogen peroxide and/or onto the reactor itself. Such coating may suitably be by deposition from a solution of an acidic material, or of from a reaction mixture containing the precursors thereof, or by applying the acidic material as such directly to the surface. A particularly suitable combination of solid material and support is boric acid coated onto glass.

Besides acidity and suitability for adsorption of hydrogen peroxide thereon, various other features contribute to suitability in the material providing the solid surface. Such features include a lack of undue reactivity with nitric acid or water formed in the course of the process and a lack of undue solubility in water and any organic materials, such as excess substrate, which may be present to the extent that the acidic solid surface should not be dissolved away. Such features are possessed by the materials disclosed above.

Preferably the invention is performed in a single composite reaction stage hydrogen peroxide, alkane and nitrogen dioxide being introduced into one end of a reactor, for example a tube or packed flow reactor, through which a flow of the resulting gas mixture, comprising reactants, reaction products and diluent if used, is maintained. The reactants and diluent may be introduced into the reactor separately although it is found to be particularly suitable to introduce the organic compound and nitrogen dioxide as one gaseous flow and the hydrogen peroxide and diluent, if any, as another gaseous flow.

Preferably the reactant streams are introduced axially into the reactor. Particularly preferably the hydrogen peroxide stream is introduced into an established stream of the organic compound and nitrogen dioxide. Preferably, the said introduction is co-current to and axially with the stream of organic compound and nitrogen dioxide. As a result, the mixed streams of reactants and diluent flow progressively along the reactor towards the exit while the introduction of the organic compound is in operation. In the single stage type of process it is preferred to use a greater concentration of organic compound than of nitrogen dioxide so as to reduce the proportion of hydroxyl radicals consumed by reaction with the nitrogen dioxide. Very suitably, the ratio of the concentration of the organic compound to that of the nitrogen dioxide is at least 10 to 1 for example possibly, up to 100 to 1 or even up to 500:1 or even greater.

When operating the process in a single stage the concentration of nitrogen dioxide is, preferably, greater than that of the hydrogen peroxide. Preferably, the ratio of the concentration of the nitrogen dioxide to that of the hydrogen peroxide is at least 10 to 1, for example possibly up to 50:1 or even greater.

Examples of suitable concentrations for the organic compound, the nitrogen dioxide and the hydrogen peroxide are respectively from 0.2 to $15\times10^{-2}$ for example $1.6\times10^{-2}$ mol $dm^{-3}$, 0.5 to $50\times10^{-5}$ for example $5\times10^{-5}$ mol $dm^{-3}$ and 0.2 to $25\times10^{-6}$ for example $2.3\times10^{-6}$ mol $dm^{-3}$.

Whatever the method used to bring the reactants into contact the process is operated at a temperature, of preferably, less than 200°C and, particularly preferably, less than 100°C. Very suitably the process is operated at a temperature of at least 0°C. Operation at superatmospheric pressure is not excluded from the scope of the invention although, preferably, a pressure of from $10^3Nm^{-2}$ to $93.3\times10^3Nm^{-2}$ may be used.

A gaseous diluent inert to the reaction may be used as required to adjust the concentrations of the reactants. Suitably the gaseous diluent is an inert gas or nitrogen. The use of additives to facilitate the operation of the nitration process is not excluded as is apparent from the development of the invention described below.

The product of the invention as above specifically described tends to be a mixture of two or all of nitro-compounds ($RNO_2$), nitrates ($RONO_2$) and nitrites (RONO), where R is the radical of the organic compound used as substrate the proportions depending on the particular process conditions employed, and usually contains a residual quantity of the organic substrate where, as is preferred, an excess thereof has been used.

In such product nitro-compound is generally present in a substantial quantity. A typical product of said invention as applied to ethane comprised 3.4% $C_2H_5ONO_2$/39% $C_2H_5ONO_2$/43% $C_2H_5NO_2$.

According to a further development of the present invention the composition of the product may be directed substantially wholly, or to a desired extent, to the nitrate with a decreased quantity of nitrocompound and nitrite by the use of an additive comprising molecular oxygen which additive would have been expected to direct a nitration reaction to an increased proportion of nitrocompound. The nitrate so produced usually comprises a mixture of the possible isomers.

The composition of the final product tends to relate to the quantity of oxygen additive used. Preferably, the oxygen additive is present in at least 1.75%, particularly preferably at least 5% and very suitably at least 6% as a proportion of the entire gaseous input into the reaction on a molar basis. There is no upper limit although for practical considerations the oxygen additive may suitably be present in less than 50% and preferably less than 45% on the same volume basis. It may be desired to control the composition of the product at some point short of the maximum production of alkyl nitrates in which case an appropriate quantity of oxygen between, for example, 1% and 10% may be selected on the same molar basis.

Apart from the use of oxygen as an additive this development is carried out in the same manner as that set out in detail in the preceding pages of this description.

It is a feature of the invention that the product may be condensed from the gas phase and directly used. One application for the product of the present invention is as an additive to fuel mixtures for internal combustion engines whether of the spark ignition or of the compression ignition type and the present invention therefore further provides such a fuel additive and a process for operating an internal combustion engine utilising such a fuel additive. Alternatively, the residual organic substrate or some of it may be removed for example by distillation and, if desired, the components of the mixture separated.

The invention will now be illustrated by means of Examples 1 to 25.

Examples 1 to 12

A tube reactor of glass having a coating of boric acid on the inner wall surface and painted black on the external surface to exclude light was used. The reactor was equipped with a cooling jacket, an inlet axially positioned at one end, and an inlet conduit entering the reactor radially shortly downstream of the first mentioned inlet but bent in the downstream direction at its end to coincide with the reactor axis, and an outlet at the end distant from the inlet. Additionally the reactor was equipped with means to control and monitor the pressure therein.

In operation a mixture of vapour of the organic compound to be nitrated, oxygen if present, and $NO_2$ was passed into the reactor through the axial inlet and a mixture of hydrogen peroxide vapour and nitrogen was passed into the resulting stream of this vapour mixture through the inlet conduit. The gases were passed through the reactor, removed through the outlet and passed to a trap and analysis system. The gases resulting from the reaction were examined by gas chromatography. The individual components of the mixture were identified by comparison of their retention times with pure compounds. The concentrations of the individual components of the mixture were derived by comparing the various peak areas with a calibration graph constructed using known concentrations of the components.

A series of experiments (Examples 1—12) was conducted using a reactor tube pressure of $39.9\times10^3 NM^{-2}$ and a temperature of 298+/−1°K (25°C+/−1°C). In Examples 1—12 the organic compound to be nitrated was ethane. The concentration of $NO_2$ was varied against a constant concentration of ethane of $1.5\times10^{-2}$ mol $dm^{-3}$ and of hydrogen peroxide of $2.3\times10^{-6}$ mol $dm^{-3}$. The residence time of the mixed reactants in the reactor was 70 sec. No oxygen additive was used. The yields of ethyl nitrite, ethyl nitrate and nitroethane are shown respectively in columns 2, 3 and 4 following Table 1 for a number of different $NO_2$ concentrations shown in Column 1.

TABLE 1

| Example | 1 $10^3[NO_2]$ / $[C_2H_6]$ | 2 $10^7[C_2H_5ONO]$ mol $dm^{-3}$ | 3 $10^7[C_2H_5ONO_2]$ mol $dm^{-3}$ | 4 $10^7[C_2H_5NO_2]$ mol $dm^{-3}$ | 5 $10^{-6}([C_2H_5ONO_2]$ / $+[C_2H_5NO_2])^{-1}$ $dm^3$ $mol^{-1}$ |
|---|---|---|---|---|---|
| 1 | 1.36 | 0.35+/−0.01 | 3.54+/−0.18 | 4.16+/−0.25 | 1.24+/−0.08 |
| 2 | 1.52 | 0.42+/−0.02 | 4.12+/−0.22 | 5.42+/−0.32 | 1.05+/−0.06 |
| 3 | 3.54 | 0.35+/−0.01 | 4.09+/−0.22 | 4.46+/−0.27 | 1.16+/−0.07 |
| 4 | 3.54 | 0.34+/−0.01 | 4.47+/−0.24 | 4.63+/−0.33 | 1.10+/−0.06 |
| 5 | 3.56 | 0.31+/−0.01 | 3.88+/−0.21 | 3.40+/−0.21 | 1.37+/−0.08 |
| 6 | 3.82 | * | 3.62+/−0.19 | 4.12+/−0.24 | 1.29+/−0.08 |
| 7 | 3.88 | * | 3.79+/−0.20 | 4.04+/−0.21 | 1.28+/−0.08 |
| 8 | 7.86 | * | 2.91+/−0.16 | 3.04+/−0.18 | 1.68+/−0.09 |
| 9 | 7.86 | * | 2.89+/−0.15 | 2.97+/−0.16 | 1.71+/−0.10 |
| 10 | 10.14 | * | 2.78+/−0.15 | 3.17+/−0.19 | 1.68+/−0.09 |
| 11 | 10.19 | * | 2.62+/−0.16 | 2.87+/−0.17 | 1.82+/−0.10 |
| 12 | 10.28 | * | 2.92+/−0.16 | 2.96+/−0.18 | 1.70+/−0.10 |

* Product not detected.

A plot of the values in Column 5 of the Table (which neglect the small and sometimes undetectable quantity of ethyl nitrite for consistency) versus those in column 1 is found to be linear and to give an intercept of $(1.01+/-0.07)\times10^6$ $dm^3$ $mol^{-1}$ corresponding to $[OH]_o^{-1}$ which gives a value for $[OH]_o$; the initial concentration of hydroxyl radicals; of $(9.9+/-0.6)\times10^{-7}$ mol $dm^{-3}$.

The intercept was derived from a least mean squares analysis of the data and the errors quoted represent one standard deviation.

The value of $[OH]_o$ thus obtained indicates an approximate 43% conversion of the $H_2O_2$ into hydroxyl radicals. This compares with the conversions of up to 82.6% confirmed for the generation of hydroxyl radicals from $H_2O_2$ catalysed by boric acid surfaces.

Relative to the value $[OH]_o$ derived above the average yields shown in Table 1 above for low $NO_2$ concentrations is as follows:

| | |
|---|---|
| $C_2H_5ONO$ | 3.4+/−0.5% |
| $C_2H_5ONO_2$ | 39+/−5% |
| $C_2H_5NO_2$ | 43+/−8% |

representing an 85.4+/−13.5% total yield of these compounds relative to the value $[OH]_o$.

Example 13

A further experiment was conducted using the same techniques (with one exception noted hereafter) as described above in which the nitration of n-pentane was investigated. The concentrations of the reactants introduced into the reactor were as follows:

| | |
|---|---|
| $n\text{-}C_5H_{12}$ | $6.4\times10^{-4}$ mol $dm^{-3}$ |
| $NO_2$ | $5.5\times10^{-5}$ mol $dm^{-3}$ |
| $H_2O_2$ | $2.3\times10^{-6}$ mol $dm^{-3}$ |
| $N_2$ | $1.4\times10^{-2}$ mol $dm^{-3}$ (diluent) |

A reactor pressure of 39.9 kPa and temperature of 298+/−1°K (25°C+/−1°C) were used. The concentrations of nitrated products so obtained, averaged from these determinations, was:—

$$\frac{10^7[C_5H_{11}ONO]}{\text{mol dm}^{-3}}=3.6+/-0.3$$

$$\frac{10^7[C_5H_{11}ONO_2]}{\text{mol dm}^{-3}}=1.1+/-0.1$$

$$\frac{10^7[C_5H_{11}NO_2]}{\text{mol dm}^{-3}}=0.9+/-0.1$$

On the assumption that the same yield of hydroxyl radicals would have been obtained as in the preceding experiments; since these two series of experiments were conducted consecutively, the yield of the above compounds based on the value of $[OH]_o$ was:

| | | |
|---|---|---|
| $C_5H_{11}ONO$ | = | 36+/−5% |
| $C_5H_{11}ONO_2$ | = | 11+/−1% |
| $C_5H_{11}NO_2$ | = | 9+/−1% |

giving a total yield of 56%+/−7%.

In conducting these experiments, but not the preceding experiments, a proportion of residual n-$C_5H_{12}$/ was removed by distillation to facilitate the determination of the product. This could be a factor in the relatively low yield obtained since nitrated products could have been removed.

Examples 14—25

Further experiments were conducted using the same techniques described for Examples 1—12 using normal pentane as the organic compound to be nitrated. In these experiments the concentration of $NO_2$ was $5.5\times10^{-5}$ mol dm$^{-3}$ the concentration of normal pentane was $1.3\times10^{-3}$ mol dm$^{-3}$ the concentration of hydrogen peroxide was $3.0\times10^{-6}$ mol dm$^{-3}$ and the concentration of nitrogen was $1.48\times10^{-2}$—$[O_2]$ mol dm$^{-3}$. The quantity of oxygen was varied between 0 in Example 14 to $72.4\times10^{-4}$ mol dm$^{-3}$. The flow rate of the total reactants was $6.5\times10^{-5}$ mol per second.

Table 2 sets out the %$O_2$ by volume in the carrier gas, the absolute yield, the % molar of total organic products for $C_5H_{11}ONO$, $C_5H_{11}ONO_2$ and $C_5H_{11}NO_2$ and $C_5$ Ketone+Aldehyde formed and the total absolute combined yield.

TABLE 2

| Ex. No. | $O_2$ mol $dm^{-3}\times10^4$ | % v/v | $C_5H_{11}ONO$ mol $dm^{-3}\times10^7$ | % Molar | $C_5H_{11}ONO_2$ mol $dm^{-3}\times10^7$ | % Molar |
|---|---|---|---|---|---|---|
| 14 | 0 | 0 | 3.2 | 30 | 0.14 | 1.3 |
| 15 | 1.7 | 1 | 1.3 | 14 | 3.8 | 41 |
| 16 | 9.9 | 6 | 0.07 | 0.7 | 8.9 | 85 |
| 17 | 15.4 | 9.5 | 0.13 | 1.5 | 7.2 | 86.9 |
| 18 | 26.3 | 16 | 0.35 | 3.2 | 9.5 | 86.7 |
| 19 | 31.5 | 19.5 | 0.26 | 2.4 | 9.5 | 88.6 |
| 20 | 34.8 | 22 | 0.19 | 2.3 | 7.2 | 86.8 |
| 21 | 37.8 | 23 | 0.21 | 1.7 | 10.9 | 88.2 |
| 22 | 50.2 | 31 | 0.27 | 2.7 | 8.7 | 88.2 |
| 23 | 57.4 | 36 | 0.11 | 1.1 | 9.0 | 88.5 |
| 24 | 65.8 | 41 | 0.17 | 1.6 | 9.5 | 89.3 |
| 25 | 72.5 | 45 | 0.07 | 0.6 | 9.6 | 88.9 |

TABLE 2 Continued

| Ex. No. | $C_5H_{11}NO_2$ mol $dm^{-3}\times10^7$ | % Molar | Ketone+aldehyde mol $dm^{-3}\times10^7$ | % Molar | Total yield mol $dm^{-3}\times10^7$ |
|---|---|---|---|---|---|
| 14 | 7.3 | 68.7 | not detected | | 10.64 |
| 15 | 4.0 | 43.0 | 0.2 | 2.0 | 9.3 |
| 16 | 1.2 | 11.4 | 0.3 | 2.9 | 10.47 |
| 17 | 0.66 | 8.0 | 0.3 | 3.6 | 8.29 |
| 18 | 0.60 | 5.5 | 0.51 | 4.6 | 10.83 |
| 19 | 0.47 | 4.4 | 0.49 | 4.6 | 10.72 |
| 20 | 0.38 | 4.6 | 0.52 | 6.3 | 8.29 |
| 21 | 0.47 | 3.8 | 0.78 | 6.3 | 12.36 |
| 22 | 0.28 | 2.8 | 0.62 | 6.3 | 9.87 |
| 23 | 0.26 | 2.6 | 0.80 | 7.8 | 10.17 |
| 24 | 0.25 | 2.4 | 0.71 | 6.7 | 10.63 |
| 25 | 0.20 | 1.8 | 0.94 | 8.7 | 10.81 |

**Claims**

1. A process for the nitration of one or more organic compounds comprising reacting, in the vapour phase, the one or more organic compounds with a nitrating agent the process being characterised in that radicals of the one or more organic compounds are produced for nitration by reacting hydrogen peroxide vapour and nitrogen dioxide in the presence of a solid surface to produce hydroxyl radicals and reacting the hydroxyl radicals with a vapour of a reactive substrate mainly comprising the one or more organic compounds by forming a mixture containing the hydroxyl radicals and the vapour of the reactive substrate or containing hydrogen peroxide vapour and nitrogen dioxide, as hydroxyl radical precursors, in the

presence of the solid surface, and the vapour of the reactive substrate and in that the radicals so produced are contacted with the nitrating agent.

2. A process as claimed in claim 1 wherein the solid surface is an acidic solid surface.

3. A process as claimed in claim 1 or 2 wherein the nitrating agent is nitrogen dioxide.

4. A process as claimed in claim 1 conducted in a single stage by passing a stream comprising a mixture of hydrogen peroxide vapour, nitrogen dioxide and vapour of the one or more organic compounds through a reactor containing an acidic solid surface, the stream being in contact with the said surface, and removing from the reactor a product vapour stream comprising nitrated derivatives of the one or more organic compounds.

5. A process as claimed in claim 4 wherein a stream comprising a mixture of vapour of said one or more organic compounds and nitrogen dioxide is introduced into the reactor and hydrogen peroxide vapour is introduced into said stream within the reactor.

6. A process as claimed in any claim 4 or 5 wherein the ratios of concentrations of the one or more organic compound, the nitrogen dioxide and the hydrogen peroxide are from 0.2 to $15\times10^{-2}$:0.5 to $50\times10^{-5}$:0.2 to $25\times10^{-6}$ mol $dm^{-3}$.

7. A process as claimed in any one of claims 4 to 6 operated at a reactor temperature of from 0°C to 200°C.

8. A process as claimed in any one of claims 4 to 7 operated at a reactor pressure of from $10^3Nm^{-2}$ to $93.3\times10^3Nm^{-2}$.

9. A process as claimed in any one of claims 4 to 8 wherein a residual proportion of the said one or more organic compounds is present in the product vapour stream together with nitrated derivatives thereof and at least a proportion of said residual quantity is removed from said derivatives.

10. A process as claimed in any preceding claim wherein the solid surface comprises one or more solid acids or one or more liquid or gaseous acids adsorbed on a solid surface or one or more solid acidic oxides or mixed oxides.

11. A process as claimed in any preceding claim wherein the one or more organic compounds are selected from aliphatic compounds belonging to the alcohols, ketones, aldehydes, ethers or alkanes and aromatic derivatives thereof.

12. A process as claimed in claim 11 wherein the one or more organic compounds are selected from straight or branched chain alkanes containing from 1 to 10 carbon atoms.

13. A process as claimed in any preceding claim wherein the reactive substrate consists of the one or more organic compounds.

14. A process as claimed in any preceding claim carried out in the presence of an additive comprising molecular oxygen.

15. A process as claimed in claim 14 wherein the molecular oxygen is present in from 1.75% to 50% by volume of the entire gaseous input into the process.

16. The product of a process as claimed in any one of claims 1 to 13 comprising a mixture of a residual quantity of one or more organic compounds with the corresponding nitro-compound, nitrate and nitrite.

17. The product of a process as claimed in claim 14 or 15 comprising a mixture of a residual quantity of said one or more organic compounds and nitrated derivatives thereof which derivatives preponderate in the nitrate.

**Patentansprüche**

1. Ein Verfahren zur Nitrierung einer oder mehrerer organischer Verbindungen umfassend die Umsetzung der einen oder mehreren organischen Verbindung(en) mit einem Nitriermittel in der Dampfphase, welches Verfahren dadurch gekennzeichnet ist, daß die Radikale einen oder mehreren organischen Verbindung(en) für die Nitrierung erhalten werden durch Umsetzen von Wasserstoffperoxid-Dampf und Stickstoffdioxid in Gegenwart einer festen Oberfläche zwecks Erhalt von Hydroxylgruppen und durch Umsetzen der Hydroxylgruppen mit dem Dampf eines reaktiven Substrats, das im wesentlichen die eine oder mehrere organische Verbindung(en) enthält, durch Bilden einer Mischung enthaltend die Hydroxylgruppen und den Dampf des reaktiven Substrats oder enthaltend Wasserstoffperoxid-Dampf und Stickstoffdioxid, als Vorstufen der Hydroxylgruppen, in Gegenwart der festen Oberfläche und des Dampfes des reaktiven Substrats, und daß die so hergestellten Radikale mit dem Nitriermittel in Berührung gebracht werden.

2. Ein Verfahren wie in Anspruch 1 beansprucht, in welchem die feste Oberfläche eine saure feste Oberfläche ist.

3. Ein Verfahren wie in Anspruch 1 oder 2 beansprucht, in welchem das Nitriermittel Stickstoffdioxid ist.

4. Ein Verfahren wie in Anspruch 1 beansprucht, das in einer einzigen Verfahrensstufe durchgeführt wird, indem man einen Strom enthaltend eine Mischung aus Wasserstoffperoxid-Dampf, Stickstoffdioxid und Dampf der einen oder mehreren organischen Verbindung(en), durch einen Reaktor leitet, der eine saure feste Oberfläche enthält, wobei der Strom mit der genannten Oberfläche kontaktiert wird, und von dem Reaktor einen Dampf-Produktstrom, enthaltend nitrierte Derivate der einen oder mehreren organischen Verbindung(en) abzieht.

5. Ein Verfahren wie in Anspruch 4 beansprucht, in welchem ein Strom enthaltend eine Mischung aus Dampf der genannten einen oder mehreren organischen Verbindung(en) und Stickstoffdioxid, in den Reaktor eingeführt wird und der Wasserstoffperoxid-Dampf innerhalb des Reaktors in diesen Strom eingeleitet wird.

6. Ein Verfahren wie in Anspruch 4 oder 5 beansprucht, in welchem die Konzentrationsverhältnisse der einen oder mehreren organischen Verbindung(en), des Stickstoffdioxids und des Wasserstoffperoxids im Bereich von 0,2 bis $15\times10^{-2}$:0,5 bis $50\times10^{-5}$:0,2 bis $25\times10^{-6}$ Mol $dm^{-3}$ liegen.

7. Ein Verfahren wie in einem der Ansprüche 4 bis 6 beansprucht, das bei einer Reaktortemperatur im Bereich von 0°C bis 200°C durchgeführt wird.

8. Ein Verfahren wie in einem der Ansprüche 4 bis 7 beansprucht, das bei einem Reaktordruck von $10^3Nm^{-2}$ bis $93,3\times10^3Nm^{-2}$ durchgeführt wird.

9. Ein Verfahren wie in einem der Ansprüche 4 bis 8 beansprucht, in welchem ein Restanteil der genannten einen oder mehreren organischen Verbindung(en) zusammen mit nitrierten Derivaten davon im Dampf-Produktstrom vorhanden ist und mindestens ein Anteil der genannten Restmenge von diesen Derivaten abgetrennt wird.

10. Ein Verfahren wie in einem der vorstehenden Ansprüche beansprucht, in welchem die feste Oberfläche eine oder mehrere Säure in fester Form oder eine oder mehrere Säuren in flüssigem oder gasförmigem Zustand, die an einer festen Oberfläche adsorbiert sind, der eine oder mehrere feste saure Oxide oder gemischte Oxide enthält.

11. Ein Verfahren wie in einem der vorstehenden Ansprüche beansprucht, in welchem die eine oder mehreren organischen Verbindung(en) ausgewählt sind aus aliphatischen Verbindungen, die zu den Alkoholen, Ketonen, Aldehyden, Äthern oder Alkanen gehören und aromatischen Derivaten derselben.

12. Ein Verfahren wie in Anspruch 11 beansprucht, in welchem die eine oder mehreren organischen Verbindung(en) ausgewählt sind aus gerad- oder verzweigtkettigen Alkanen mit 1 bis 10 Kohlenstoffatomen.

13. Ein Verfahren wie in einem der vorstehenden Ansprüche beansprucht, in welchem das reaktive Substrat aus der einen oder mehreren organischen Verbindung(en) besteht.

14. Ein Verfahren wie in einem der vorstehenden Ansprüche beansprucht, das in Gegenwart eines molekularen Sauerstoff enthaltenden Zusatzstoffes durchgeführt wird.

15. Ein Verfahren wie in Anspruch 14 beansprucht, in welchem der molekulare Sauerstoff in einer Menge von 1,75 bis 50 Volumenprozent der gesamten in das Verfahren eingespeisten Gasmenge vorliegt.

16. Das Produkt eines Verfahrens wie in einem der Ansprüche 1 bis 13 beansprucht, umfassend eine Mischung einer Restmenge der genannten einen oder mehreren organischen Verbindung(en) mit der entsprechenden Nitroverbindung, Nitrat und Nitrit.

17. Das Produkt eines Verfahrens wie in Anspruch 14 oder 15 beansprucht, umfassend eine Mischung einer Restmenge der genannten einen oder mehreren organischen Verbindung(en) und nitrierte Derivate davon, wobei Derivate in Form des Nitrats überwiegen.

**Revendications**

1. Procédé de nitration d'un ou plusieurs composés organiques comprenant la mise en réaction, en phase vapeur, du ou des composés organiques avec un agent de nitration, le procédé étant caractérisé en ce que l'on produit pour la nitration des radicaux du ou des composés organiques en faisant réagir de la vapeur de peroxyde d'hydrogène et du dioxyde d'azote en présence d'une surface solide afin de produire des radicaux hydroxyle et en faisant réagir les radicaux hydroxyle avec la vapeur d'un substrat réactif comprenant principalement le ou les composés organiques en formant un mélange contenant les radicaux hydroxyle et la vapeur du substrat réactif ou bien contenant de la vapeur de peroxyde d'hydrogène et du dioxyde d'azote, comme précurseurs de radical hydroxyle, en présence de la surface solide, et la vapeur du substrat réactif et en ce que les radicaux ainsi produits sont mis en contact avec l'agent de nitration.

2. Procédé selon la revendication 1 caractérisé en ce que la surface solide consiste en une surface solide acide.

3. Procédé suivant l'une des revendications 1 ou 2 caractérisé en ce que l'agent de nitration est du dioxyde d'azote.

4. Procédé suivant la revendication 1 caractérisé en ce qu'il est exécuté en une étape unique en faisant passer un courant comprenant un mélange de vapeur de peroxyde d'hydrogène, de dioxyde d'azote et de la vapeur du ou des composés organiques à travers un réacteur contenant une surface solide acide, le courant étant en contact avec ladite surface, et en retirant du réacteur un courant de vapeur d'un produit comprenant des dérivés azotés du ou des composés organiques.

5. Procédé suivant la revendication 4 caractérisé en ce qu'on introduit dans le réacteur un courant comprenant un mélange de vapeur du ou des composés organiques et de dioxyde d'azote et en ce qu'on introduit de la vapeur de peroxyde d'hydrogène dans ledit courant, à l'intérieur du réacteur.

6. Procédé suivant l'une quelconque des revendications 4 ou 5 caractérisé en ce que les rapports des concentrations en le ou les composés organiques, en dioxyde d'azote et en peroxyde d'hydrogène sont de 0,2 à $15\times10^{-2}$ mol $dm^{-3}$/0,5 à $50\times10^{-5}$ mol $dm^{-3}$/0,2 à $25\times10^{-6}$ mol $dm^{-3}$.

7. Procédé suivant l'une quelconque des revendications 4 à 6 caractérisé en ce qu'il est effectué à une température du réacteur comprise entre 0°C et 200°C.

8. Procédé suivant l'une quelconque des revendications 4 à 7 caractérisé en ce qu'il est effectué à une pression du réacteur comprise entre $10^3 Nm^{-2}$ et $93,3\times10^3\ Nm^{-2}$.

9. Procédé suivant l'une quelconque des revendications 4 à 8 caractérisé en ce qu'une proportion résiduelle du ou des composés organiques est présente dans le courant de vapeur du produit en même temps que des dérivés nitrés de ceux-ci et en ce qu'au moins une proportion de ladite quantité résiduelle est séparée desdits dérivés.

10. Procédé suivant l'une quelconque des revendications précédentes caractérisé en ce que la surface solide comprend un ou plusieurs acides solides ou un ou plusieurs acides liquides ou gazeux adsorbés sur une surface solide ou encore un ou plusieurs oxydes acides ou oxydes mixtes solides.

11. Procédé suivant l'une quelconque des revendications précédentes caractérisé en ce que le ou les composés organiques sont choisis parmi des composés aliphatiques appartenant aux alcools, aux cétones, aux aldéhydes, aux éthers ou aux alcanes et parmi des dérivés aromatiques de ceux-ci.

12. Procédé suivant la revendication 11 caractérisé en ce que le ou les composés organiques sont choisis parmi les alcanes à chaîne droite ou à chaîne ramifiée contenant de 1 à 10 atomes de carbone.

13. Procédé suivant l'une quelconque des revendications précédentes caractérisé en ce que le substrat réactif est constitué par le ou les composés organiques.

14. Procédé suivant l'une quelconque des revendications précédentes caractérisé en ce qu'il est réalisé en présence d'un additif comprenant de l'oxygène moléculaire.

15. Procédé suivant la revendication 14 caractérisé en ce que l'oxygène moléculaire est présent à raison de 1,75% à 50% en volume de la quantité totale de gaz introduite.

16. Le produit d'un procédé tel que revendiqué dans l'une quelconque des revendications 1 à 13 comprenant un mélange d'une quantité résiduelle d'un ou plusieurs composés organiques et du composé nitro, du nitrate et du nitrite correspondants.

17. Le produit d'un procédé tel que revendiqué dans l'une des revendications 14 ou 15 comprenant un mélange d'une quantité résiduelle d'un ou plusieurs composés organiques et de dérivés nitrés de ceux-ci, dérivés qui consistent de manière prépondérante en nitrate.